# EUROPEAN PATENT APPLICATION

(11) **EP 4 502 168 A1**
(43) Date of publication of application: **05.02.2025**
(21) Application number: 23775120.1
(22) Date of filing: 24.03.2023
(51) Int. Cl.: C12P 19/00, C12N 1/15, C12N 1/19, C12N 1/21, C12N 15/31, C12N 15/54

(54) **PRODUCTION METHOD FOR LACTODIFUCOTETRAOSE (LDFT)**

(30) Priority: 25.03.2022 JP 2022050799
(71) Applicant: KIRIN HOLDINGS KABUSHIKI KAISHA, Nakano-ku, Tokyo 164-0001 (JP)
(72) Inventor: SUGITA Tomotoshi, Tokyo 164-0001 (JP); SANPEI Sotaro, Tokyo 164-0001 (JP)
(74) Representative: Grünecker Patent- und Rechtsanwälte PartG mbB
(86) International application number: PCT/JP2023/012041
(87) International publication number: WO 2023/182527

(57) **Abstract**

The purpose of the present invention is to provide a microorganism excelling in LDFT productivity. The present invention relates to a microorganism in which the activity of one among [1] a protein composed of an amino acid sequence represented by sequence number 8, and a mutant protein or homologous protein thereof and [4] a protein composed of an amino acid sequence represented by sequence number 2, and a mutant protein or homologous protein thereof is heightened, and which has improved lactodifucotetraose (LDFT) productivity compared to a parent strain.

## Description

### TECHNICAL FIELD

The present invention relates to a method for producing lactodifucotetraose (LDFT).

### BACKGROUND ART

Human milk oligosaccharides (HMO) are considered to be useful for prebiotic effects, regulation of the intestinal environment and immune function, defense against infection, and cognitive development in infants (Non Patent Literature 1).

Lactodifucotetraose (hereinafter, referred to as LDFT) is a type of HMO, and is a tetrasaccharide fucosylated oligosaccharide in which fucose is bonded to 2-position of galactose and 3-position of glucose in lactose via an α1,2 or α1,3 bond, respectively. LDFT is an HMO that has both structures of 2'-fucosyllactose (hereinafter, referred to as 2'FL) and 3-fucosyllactose (hereinafter, referred to as 3FL), which are also known as fucosylated oligosaccharides.

It has been reported that in human milk, LDFT is contained at about 0.5 g/L in a transitional milk from colostrum to whole milk (Non-Patent Literature 2). The functionality of LDFT is known to include a strong antibacterial effect against Group B Streptococcus (Non Patent Literature 3), but the functionality research is still in progress.

A widely known method for producing LDFT is a microbial fermentation method using a microorganism expressing fucosyltransferase and using lactose as a substrate. It has been reported that in the microbial fermentation method, LDFT can be produced efficiently by using α1,2-fucosyltransferase alone or a combination of α1,2-fucosyltransferase and α1,3-fucosyltransferase as the fucosyltransferase (Patent Literatures 1 and 2, and Non Patent Literature 4).

Examples of the α1,2-fucosyltransferase used alone in the above-described microbial fermentation method include FutC derived from Helicobacter pylori (Patent Literature 1).

Examples of the α1,2-fucosyltransferase when α1,2-fucosyltransferase and α1,3-fucosyltransferase are used in combination in the above-described microbial fermentation method include WbgL derived from Escherichia coli O126 (Patent Literature 2 and Non Patent Literature 4).

Examples of the α1,3-fucosyltransferase when α1,2-fucosyltransferase and α1,3-fucosyltransferase are used in combination in the above-described microbial fermentation method include CafA derived from Bacteroides fragilis, CafC derived from Bacteroides nordii, CafF derived from Akkermansia muciniphila (all of which are disclosed in Patent Literature 2), or Hp3/4FT derived from Helicobacter pylori UA948 (Non Patent Literature 4).

Non Patent Literature 4 discloses a process for producing LDFT in a single flow from lactose via 2'FL using α1,2-fucosyltransferase WbgL, which is substrate specific for lactose and cannot use 3FL, which is an intermediate of LDFT, as a substrate, and α1,3-fucosyltransferase Hp3/4FT, which uses 2'FL as a substrate.

### CITATION LIST

### PATENT LITERATURE

Patent Literature 1: WO2015/032413
Patent Literature 2: JP2017-527311A

### NON-PATENT LITERATURE

Non Patent Literature 1: Trends in Food Science & Technology, 118 (2021) 374-387
Non Patent Literature 2: Nutrients, 2021, 13 (8) 2737
Non Patent Literature 3: Accounts of Chemical Research, 2019, 52 (3) 760-768
Non Patent Literature 4: Metabolic Engineering, 66 (2021) 12-20

### SUMMARY OF INVENTION

### TECHNICAL PROBLEM

However, the above method described in Non Patent Literature 4 has problems that Hp3/4FT also reacts with lactose, resulting in accumulation of large amounts of 3FL as a by-product, and that 2'FL remains as a by-product due to insufficient conversion activity of Hp3/4FT to LDFT.

In order to produce LDFT more efficiently, it is required to search for α1,2-fucosyltransferases and α1,3-fucosyltransferases that can recognize an intermediate 2'FL or 3FL as a substrate in the same manner as lactose and can rapidly convert 2'FL or 3FL to LDFT with high efficiency.

Therefore, an object of the present invention is to provide a microorganism having excellent productivity of LDFT.

### SOLUTION TO PROBLEM

The present inventors have found that a microorganism having an enhanced activity of a protein consisting of a specific amino acid sequence has improved productivity of LDFT as compared with a parent strain, and have completed the present invention.

That is, the present invention is as follows.
1. A microorganism having enhanced activities of a protein according to any one of the following [1] to [3] and a protein according to any one of the following [4] to [6] and improved productivity of lactodifucotetraose (LDFT) as compared with a parent strain,
   [1] a protein consisting of the amino acid sequence represented by SEQ ID NO: 8,
   [2] a mutant protein having an α1,2-fucosyltransferase activity and consisting of an amino acid sequence in which 1 to 20 amino acids are deleted, substituted, inserted, or added in the amino acid sequence represented by SEQ ID NO: 8,
   [3] a homologous protein having an α1,2-fucosyltransferase activity and consisting of an amino acid sequence having an identity of 80% or more with the amino acid sequence represented by SEQ ID NO: 8,
   [4] a protein consisting of the amino acid sequence represented by SEQ ID NO: 2,
   [5] a mutant protein having an α1,3-fucosyltransferase activity and consisting of an amino acid sequence in which 1 to 20 amino acids are deleted, substituted, inserted, or added in the amino acid sequence represented by SEQ ID NO: 2, and
   [6] a homologous protein having an α1,3-fucosyltransferase activity and consisting of an amino acid sequence having an identity of 80% or more with the amino acid sequence represented by SEQ ID NO: 2.
2. A method for producing LDFT, including: preparing the microorganism according to the above 1; and producing LDFT in a culture using the microorganism.
3. A method for producing an LDFT crystal from a culture obtained by culturing the microorganism according to the above 1, the method including the following steps (i) to (iii):
   (i) a step of centrifuging the culture to obtain a supernatant from which bacteria cells are removed;
   (ii) a step of subjecting the supernatant obtained in step (i) to cation exchange and anion exchange to obtain a solution by removing ions from the supernatant; and
   (iii) a step of obtaining an LDFT crystal from the solution obtained in step (ii).

### ADVANTAGEOUS EFFECTS OF INVENTION

The microorganism according to the present invention has enhanced activities of two proteins consisting of specific amino acid sequences. One of the two proteins has an α1,2-fucosyltransferase activity capable of recognizing 3FL, which is an intermediate of LDFT, as a substrate in the same manner as lactose and rapidly converting 3FL into LDFT with high efficiency. The other one of the two proteins has an α1,3-fucosyltransferase activity capable of recognizing 2'FL, which is an intermediate of LDFT, as a substrate in the same manner as lactose and rapidly converting 2'FL into LDFT with high efficiency. By enhancing the activities of the two proteins, the microorganism according to the present invention can prevent intermediates 2'FL and 3FL from remaining as by-products, thereby improving LDFT productivity.

### BRIEF DESCRIPTION OF DRAWINGS

[FIG. 1] FIG. 1 shows a biosynthetic pathway from lactose to LDFT in one embodiment of the present invention.
[FIG. 2] FIG. 2 shows a carbohydrate analysis result of an LDFT crystal obtained in Example 7, in which a vertical axis represents an intensity, and a horizontal axis represents an analysis time.
[FIG. 3] FIG. 3 shows an oak ridge thermal ellipsoid plot (ORTEP) diagram of an LDFT•7.2 hydrate crystal.

### DESCRIPTION OF EMBODIMENTS

### <Microorganism>

A microorganism according to present invention has enhanced activities of a protein according to any one of the following [1] to [3] and a protein according to any one of the following [4] to [6] and improved productivity of lactodifucotetraose (LDFT) as compared with a parent strain,
[1] a protein consisting of the amino acid sequence represented by SEQ ID NO: 8,
[2] a mutant protein having an α1,2-fucosyltransferase activity and consisting of an amino acid sequence in which 1 to 20 amino acids are deleted, substituted, inserted, or added in the amino acid sequence represented by SEQ ID NO: 8,
[3] a homologous protein having an α1,2-fucosyltransferase activity and consisting of an amino acid sequence having an identity of 80% or more with the amino acid sequence represented by SEQ ID NO: 8,
[4] a protein consisting of the amino acid sequence represented by SEQ ID NO: 2,
[5] a mutant protein having an α1,3-fucosyltransferase activity and consisting of an amino acid sequence in which 1 to 20 amino acids are deleted, substituted, inserted, or added in the amino acid sequence represented by SEQ ID NO: 2, and
[6] a homologous protein having an α1,3-fucosyltransferase activity and consisting of an amino acid sequence having an identity of 80% or more with the amino acid sequence represented by SEQ ID NO: 2.

The protein consisting of the amino acid sequence represented by SEQ ID NO: 8 is α1,2-fucosyltransferase HMFT derived from a Helicobacter mustelae ATCC 43772 strain, which will be described later in Examples.

The protein consisting of the amino acid sequence represented by SEQ ID NO: 2 is cBrFucT derived from Bacteroides reticulotermitis and Bacteroides fragilis, which will be described later in Examples.

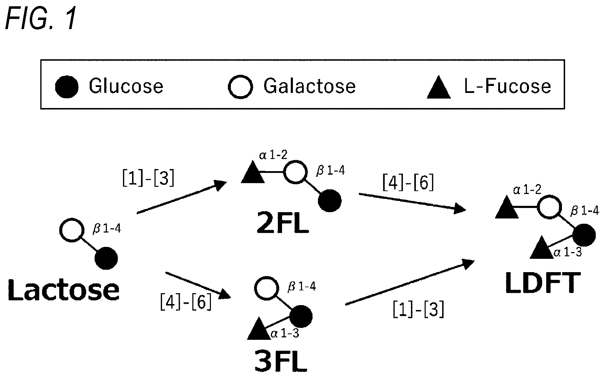

FIG. 1 shows a biosynthetic pathway from lactose to LDFT in one embodiment of the present invention.

The above proteins [1] to [3] can recognize an intermediate 3FL as a substrate in the same manner as lactose, and have an α1,2-fucosyltransferase activity capable of catalyzing a biosynthetic reaction of (lactose → 2'FL) and a biosynthetic reaction of (3FL → LDFT).

The above proteins [4] to [6] can recognize an intermediate 2FL as a substrate in the same manner as lactose, and have an α1,3-fucosyltransferase activity capable of catalyzing a biosynthetic reaction of (lactose → 3FL) and a biosynthetic reaction of (2'FL → LDFT).

Therefore, the microorganism according to the present invention has enhanced activities of a protein according to any one of the following [1] to [3] and a protein according to any one of the following [4] to [6], thereby preventing intermediates 2'FL and 3FL from remaining as by-products and rapidly converting 2'FL or 3FL into LDFT with high efficiency.

In the present description, the α1,2-fucosyltransferase activity refers to an activity of transferring a fucose residue from a donor substrate GDP-fucose to a hydroxyl group of N-acetylglucosamine in an acceptor molecule via an α1,2-bond to generate a fucose-containing carbohydrate.

In the present embodiment, lactose and 3FL are preferred as the acceptor molecule for α1,2-fucosyltransferase. When the acceptor molecule is lactose, the fucose-containing carbohydrate is preferably 2FL. When the acceptor molecule is 3FL, the fucose-containing carbohydrate is preferably LDFT.

It can be confirmed by, for example, the following method that the above mutant protein or homologous protein has an α1,2-fucosyltransferase activity.

First, a recombinant DNA comprising a DNA encoding the above mutant protein or homologous protein whose activity is to be confirmed is prepared by a method to be described later. Next, a transformant having an activity of the protein higher than that of a parent strain is prepared by transforming the parent strain with the recombinant DNA, and amounts of fucose-containing carbohydrates produced and accumulated in culture solutions of the parent strain and the transformant are compared to confirm. Specific examples of the fucose-containing carbohydrate include 2FL and LDFT.

In the present description, the α1,3-fucosyltransferase activity refers to an activity of transferring a fucose residue from a donor substrate GDP-fucose to a hydroxyl group of N-acetylglucosamine in an acceptor molecule via an α1,3-bond to generate a fucose-containing carbohydrate.

In the present embodiment, lactose and 2'FL are preferred as the acceptor molecule for α1,3-fucosyltransferase. When the acceptor molecule is lactose, the fucose-containing carbohydrate is preferably 3FL. When the acceptor molecule is 2'FL, the fucose-containing carbohydrate is preferably LDFT.

It can be confirmed by, for example, the following method that the above mutant protein or homologous protein has an α1,3-fucosyltransferase activity.

First, a recombinant DNA comprising a DNA encoding the above mutant protein or homologous protein whose activity is to be confirmed is prepared by a method to be described later. Next, a transformant having an activity of the protein higher than that of a parent strain is prepared by transforming the parent strain with the recombinant DNA, and amounts of fucose-containing carbohydrates produced and accumulated in culture solutions of the parent strain and the transformant are compared to confirm. Specific examples of the fucose-containing carbohydrate include 3FL and LDFT.

In the present description, the mutant protein refers to a protein obtained by artificially deleting or substituting an amino acid residue of an original protein or inserting or adding an amino acid residue in the protein.

The expression "an amino acid is deleted, substituted, inserted, or added in the mutant protein of the above [2] and [5]" may mean that preferably 1 to 20 amino acids are deleted, substituted, inserted, or added at any position in the same sequence. The number of amino acids to be deleted, substituted, inserted, or added is preferably 1 to 20, more preferably 1 to 10, further preferably 1 to 8, and most preferably 1 to 5.

The amino acid to be deleted, substituted, inserted, or added may be of a natural type or a non-natural type. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

In the mutant protein of the above [2]and [5], examples of the amino acid residue to be substituted include an asparagine residue at position 17.

In the present description, the homologous protein is a protein whose encoding gene is thought to have the same evolutionary origin as a gene encoding an original protein due to similarity in structure and function with the original protein, and is a protein possessed by organisms in nature.

Examples of the homologous protein include an amino acid sequence having an identity of preferably 90% or more, more preferably 93% or more, further preferably 95%, and particularly preferably 97% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST and Gapped BLAST programs are used, default parameters for each program are used. A specific method for the analysis methods is well known.

In the present description, the term "parent strain" refers to an original strain to be subjected to genetic modification, transformation, and the like.

In the present description, the parent strain is preferably a prokaryote or a yeast strain, more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and most preferably a prokaryote such as Escherichia coli MG1655, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 codon plus (manufactured by Stratagene Corporation), Escherichia coli W3110S3GK (NBRC114657), Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC 14066, Corynebacterium ammoniagenes, Corynebacterium glutamicum ATCC 13032, Corynebacterium glutamicum ATCC 14067, Corynebacterium glutamicum ATCC 13869, Corynebacterium acetoacidophilum ATCC 13870, Microbacterium ammoniaphilum ATCC 15354, or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The parent strain may be a wild strain as long as it is a microorganism that produces GDP-fucose and/or lactose. When a wild strain does not have an ability to produce GDP-fucose and/or lactose, the wild strain may be a bred strain to which an ability to supply GDP-fucose and/or lactose is artificially endowed.

Examples of the microorganism that can be used as a parent strain include the following 1) and 2).
1) A microorganism having an artificially endowed or enhanced ability to supply GDP-fucose, which is a donor substrate for α1,2-fucosyltransferase/α1,3-fucosyltransferase
2) A microorganism having an artificially endowed or enhanced ability to supply lactose, which is a receptor carbohydrate for α1,2-fucosyltransferase/α1,3-fucosyltransferase

This will be described below.

For 1) a microorganism having an artificially endowed or enhanced ability to supply GDP-fucose, which is a donor substrate for α1,2-fucosyltransferase/α1,3-fucosyltransferase, a parent strain is preferably a microorganism having an artificially endowed or enhanced ability to supply GDP-fucose, which is a reaction substrate for α1,2-fucosyltransferase/α1,3-fucosyltransferase. Specific examples of the method for endowing or enhancing an ability to supply GDP-fucose to a microorganism used as a parent strain include a known method such as a method using various genetic manipulations (Metabolic Engineering (2017) 41: 23-38).

Examples of the ability to supply GDP-fucose include an ability to produce GDP-fucose from a saccharide. Examples of the method for artificially endowing or enhancing an ability to supply GDP-fucose from a saccharide to a microorganism used as a parent strain include the following methods (1a) to (1d). These methods may be used alone or in combination.
(1a) A method of alleviating or releasing at least one mechanism for controlling a biosynthetic pathway for producing GDP-fucose from a saccharide
(1b) A method of enhancing expression of at least one enzyme associated with a biosynthetic pathway for producing GDP-fucose from a saccharide
(1c) A method of increasing the number of copies of at least one gene encoding an enzyme associated with a biosynthetic pathway for producing GDP-fucose from a saccharide
(1d) A method of weakening or blocking at least one metabolic pathway branching off from a biosynthetic pathway for producing GDP-fucose from a saccharide to a metabolic product other than a target substance

Specific examples of the mechanism for controlling a biosynthetic pathway for producing GDP-fucose from a saccharide include known mechanisms such as a control mechanism based on a transcription regulatory factor (e.g., RcsA) associated with control of the biosynthetic pathway. RcsA is a regulatory factor for uppreforming the entire cholanic acid biosynthetic pathway using GDP-fucose as an intermediate. As will be described later, by strengthening rcsA in a state where a pathway downstream of GDP-fucose in the cholanic acid biosynthetic pathway is blocked, a large amount of GDP-fucose can be accumulated.

Specific examples of the enzyme associated with the biosynthetic pathway for producing GDP-fucose from a saccharide include known enzymes such as a mannose-6-phosphate isomerase, a phosphomannomutase, a mannose-1-phosphate guanylyltransferase, a GDP mannose-4,6-dehydratase, and a GDP-L-fucose synthase.

Specific examples of the metabolic pathway branching off from a biosynthetic pathway for producing GDP-fucose from a saccharide to a metabolic product other than a target substance include a known metabolic pathway such as a cholanic acid biosynthetic pathway from GDP-fucose to cholanic acid. Particularly, the supply of GDP-fucose can be enhanced by blocking WcaJ, WzxC, WcaK, WcaL, or WcaM, which is a pathway downstream of GDP-fucose in the cholanic acid biosynthetic pathway.

The microorganism to be used as a parent strain may be modified to promote transfer of exogenous L-fucose crossing a cell membrane thereof. For example, by expressing or overexpressing the nucleotide sequence (Accession Number: AIZ90162) encoding FucP, uptake of exogenous L-fucose crossing the cell membrane into cells can be improved, and a fucose amount for producing GDP-fucose can be further increased.

The microorganism to be used as a parent strain may be modified such that genes fucI and/or fucK encoding L-fucose isomerase and L-fuculose kinase, respectively, are deleted, and nucleotide sequences of fucI and/or fucK are changed to irreversibly inactivate an enzyme activity of a corresponding polypeptide, or expression of fucI and/or fucK is impaired. When intracellular synthesis of FucI and/or FucK is eliminated, fucose metabolism in cells disappears, thereby allowing for increased amounts of fucose to produce GDP-fucose.

### 2) A microorganism having an artificially endowed or enhanced ability to supply lactose, which is a receptor carbohydrate for α1,2-fucosyltransferase/α1,3-fucosyltransferase

Examples of the method for artificially endowing an ability to supply lactose to a microorganism used as a parent strain include the following methods (2a) to (2e), and these methods can be used alone or in combination.
(2a) A method of alleviating or releasing at least one mechanism for decomposing lactose
(2b) A method of enhancing an expression of at least one enzyme associated with intracellular uptake of lactose
(2c) A method of increasing the number of copies of at least one gene encoding an enzyme associated with intracellular uptake of lactose
(2d) A method of weakening or blocking at least one metabolic pathway branching off from a biosynthetic pathway of lactose to a metabolic product other than a target substance
(2e) A method of selecting a cell strain having resistance to lactose analogues higher than that of a wild-type strain

Specific examples of the mechanism for decomposing lactose include known enzymes such as galactose and β-galactosidase, which catalyzes hydrolysis of lactose to produce glucose. Specific examples thereof include β-galactosidase (hereinafter, referred to as lacZ) which hydrolyzes lactose, and a decrease in supply of lactose can be prevented by losing the activity of lacZ.

Examples of the enzyme associated with intracellular uptake of lactose include known enzymes such as lactose permease. Specific examples thereof include lactose permease (hereinafter, referred to as lacY) which is associated with the intracellular uptake of lactose. lacY is a membrane protein which takes up lactose into cells. By enhancing an activity of lacY, supply of lactose can be enhanced.

The fact that the microorganism is a microorganism capable of producing GDP-fucose and/or lactose can be confirmed by culturing the microorganism in a culture medium and detecting GDP-fucose and/or lactose accumulated in a culture by using a general method such as a carbohydrate analyzer or a high performance liquid chromatograph mass spectrometer to be described later.

The microorganism to be used as a parent strain of the present invention is preferably a microorganism having an artificially endowed or enhanced ability to supply GDP-fucose and/or lactose, which is a reaction substrate for α1,2-fucosyltransferase/α1,3-fucosyltransferase. Accordingly, in one embodiment of the present invention, the parent strain is preferably a genetically modified microorganism comprising at least one nucleotide sequence selected from the nucleotide sequence encoding rcsA (Accession Number: BAA15776.1), the nucleotide sequence encoding mannose-6-phosphate isomerase (Accession Number: BAA15361.1), the nucleotide sequence encoding phosphomannomutase (Accession Number: BAA1590 1.1), the nucleotide sequence encoding mannose-1-phosphate guanylyltransferase (Accession Number: BAA15905.1), the nucleotide sequence encoding GDP mannose-4,6-dehydratase (Accession Number: BAA15909.1), the nucleotide sequence encoding GDP-L-fucose synthase (Accession Number: BAA15908.1), and the nucleotide sequence encoding lacY (Accession Number: BAE76125.1).

Particularly, the parent strain is more preferably a genetically modified microorganism comprising the nucleotide sequence encoding lacY and the nucleotide sequence encoding rcsA. In one embodiment of the present invention, the genetically modified microorganism preferably has an enhanced ability to produce GDP-fucose and/or lactose as compared with a parent strain that is not genetically modified.

A known method may be used as a method for producing a microorganism having at least one activity selected from a lacY activity and a rcsA activity or having an enhanced activity thereof. Specific examples thereof include methods using various genetic manipulations (Syst Microbiol Biomanufact, 2021, 1, 291).

As described above, the parent strain preferably has a reduced or deleted lacZ activity and/or cholanic acidsynthesis activity. Accordingly, in one embodiment of the present invention, the parent strain is preferably a genetically modified microorganism having a reduced or deleted lacZ activity and/or cholanic acid synthesis activity, and more preferably a genetically modified microorganism not comprising the nucleotide sequence encoding lacZ and/or the nucleotide sequence encoding a wcaJ, wzxC, wcaK, wcaL, or wcaM gene which is a nucleotide sequence encoding a protein associated with cholanic acid synthesis.

In one embodiment of the present invention, the genetically modified microorganism preferably has an enhanced ability to produce GDP-fucose and/or lactose as compared with a parent strain that is not genetically modified.

A known method may be used as a method for producing Escherichia coli having a reduced or deleted β-galactosidase activity and/or cholanic acid synthesis activity. Specific examples include methods using various genetic manipulations (Metabolic Engineering, 2017, 41: 23-38).

Examples of the microorganism having enhanced activities of the protein according to any one of the above [1] to [3] and the protein according to any one of the above [4] to [6] as compared with the microorganism of the parent strain include a microorganism having an increased copy number of the genes as compared with the parent strain, which is obtained by transforming the microorganism of the parent strain with a recombinant DNA containing a DNA encoding the protein.

Examples of the microorganism having an increased copy number of the genes as compared with the parent strain, which is obtained by transforming the microorganism of the parent strain with a recombinant DNA containing DNAs encoding the protein according to any one of the above [1] to [3]and the protein according to any one of the above [4] to [6], respectively, include a microorganism having an increased copy number of the genes on a chromosomal DNA by transforming the microorganism of the parent strain with the recombinant DNA containing the DNAs encoding the protein according to any one of the above [1] to [3]and the protein according to any one of the above [4] to [6], respectively, and a microorganism carrying the above gene outside of a chromosomal DNA as a plasmid DNA.

The DNA encoding the protein according to any one of the above [1] to [3] may be any DNA as long as it encodes a protein having an activity of a protein according to any one of [1] to [3]. Specific examples thereof include one DNA selected from the group consisting of the following [7] to [10].
[7] A DNA encoding the protein according to any one of the above [1] to [3]
[8] A DNA consisting of the nucleotide sequence represented by SEQ ID NO: 7
[9] A DNA hybridizing with a DNA consisting of a nucleotide sequence complementary to the nucleotide sequence represented by SEQ ID NO: 7 under stringent conditions and encoding a homologous protein having an α1,2-fucosyltransferase activity
[10] A DNA consisting of a nucleotide sequence having an identity of 95% or more, preferably 97% or more, more preferably 98% or more, and most preferably 99% or more with the nucleotide sequence represented by SEQ ID NO: 7 and encoding a homologous protein having an α1,2-fucosyltransferase activity

The DNA encoding the protein according to any one of the above [4] to [6] may be any DNA as long as it encodes a protein having an activity of the protein according to any one of [4] to [6]. Specific examples thereof include one DNA selected from the group consisting of the following [11] to [14].
[11] A DNA encoding the protein according to any one of the above [4] to [6]
[12] A DNA consisting of the nucleotide sequence represented by SEQ ID NO: 1
[13] A DNA hybridizing with a DNA consisting of a nucleotide sequence complementary to the nucleotide sequence represented by SEQ ID NO: 1 under stringent conditions and encoding a homologous protein having an α1,2-fucosyltransferase activity
[14] A DNA consisting of a nucleotide sequence having an identity of 95% or more, preferably 97% or more, more preferably 98% or more, and most preferably 99% or more with the nucleotide sequence represented by SEQ ID NO: 1 and encoding a homologous protein having an α1,2-fucosyltransferase activity

The term "hybridizing" in the above [9] and [13] means that a DNA hybridizes to a DNA having a specific nucleotide sequence or a part of the DNA. Accordingly, the DNA having a specific nucleotide sequence or a part thereof is a DNA that can be used as a probe for Northern or Southern blot analysis, or a DNA that can be used as an oligonucleotide primer for PCR analysis.

Examples of the DNA used as a probe include a DNA having at least 100 bases or more, preferably 200 bases or more, and more preferably 500 bases or more. Examples of the DNA used as a primer include a DNA having at least 10 bases or more, and preferably 15 bases or more.

A method for a DNA hybridization experiment is well known, and for example, those skilled in the art can determine hybridization conditions according to the present description. The hybridization conditions can be determined according to those described in Molecular Cloning, 4th Edition (2012), Methods for General and Molecular Bacteriology, ASM Press (1994), and Immunology methods manual, Academic press(1996) as well as many other standard textbooks.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mmol/L sodium chloride, 75 mmol/L sodium citrate), 50 mmol/L sodium phosphate (pH 7.6), a 5 × Denhardt's solution, 10% dextran sulfate, and a 20 µg/l of denatured salmon sperm DNA, and then the filter is washed in a 0.2×SSC solution at, for example, about 65°C.

Examples of the DNA capable of hybridizing under the above stringent conditions include a DNA having an identity of at least 95% or more, preferably 97% or more, more preferably 98% or more, and most preferably 99% or more with the DNA consisting of the nucleotide sequence represented by SEQ ID NO: 7 or 1 when calculated based on the parameters or the like using BLAST, FASTA, or the like.

The DNA encoding the protein of the above [1] can be obtained by, for example, Southern hybridization for a chromosomal DNA library of a microorganism, preferably a microorganism belonging to the genus Helicobacter, and more preferably a Helicobacter mustelae ATCC 43772 strain using a probe DNA that can be designed based on the nucleotide sequence represented by SEQ ID NO: 7, or PCR [PCR protocols, Academic Press (1990)] using, as a template, a chromosomal DNA of a microorganism and using a primer DNA that can be designed based on the nucleotide sequence.

Among the protein of the above [4], the DNA encoding the amino acid sequence represented by SEQ ID NO: 2 can be obtained by, for example, chimerization by fusion PCR shown in the following (i) or (ii).
(i) Chimerization, by fusion PCR, of the following three DNA fragments (i-1) to (i-3) obtained by Southern hybridization to each chromosomal DNA library using a probe that can be designed based on the nucleotide sequence of the DNA encoding the protein consisting of the amino acid sequence represented by SEQ ID NO: 2
   (i-1) A DNA fragment consisting of the nucleotide sequence at positions 1 to 342 in the nucleotide sequence represented by SEQ ID NO: 3, which is obtained by Southern hybridization to a chromosomal DNA library of a microorganism, preferably of the genus Bacteroides microorganism, and more preferably of a Bacteroides reticulotermitis JCM 10512 strain
   (i-2) A DNA fragment consisting of the nucleotide sequence at positions 607 to 954 in the nucleotide sequence represented by SEQ ID NO: 3, which is obtained by Southern hybridization to a chromosomal DNA library of a microorganism, preferably of the genus Bacteroides microorganism, and more preferably of a Bacteroides reticulotermitis JCM 10512 strain
   (i-3) A DNA fragment consisting of a nucleotide sequence obtained by codon-optimizing, for expression in Escherichia coli, a nucleotide sequence encoding the amino acid sequence at positions 127 to 214 in the amino acid sequence represented by SEQ ID NO: 6, which is obtained by Southern hybridization to a chromosomal DNA library of a Bacteroides fragilis ATCC 25285 strain
(ii) Chimerization, by fusion PCR, of the following three DNA fragments ((ii-1) to ((ii-3) obtained by PCR using a primer DNA that can be designed based on a DNA encoding the protein consisting of the amino acid sequence represented by SEQ ID NO: 4 and using each chromosomal DNA as a template
   (ii-1) A DNA fragment consisting of the nucleotide sequence at positions 1 to 342 in the nucleotide sequence represented by SEQ ID NO: 3, which is obtained by PCR using, as a template, a chromosomal DNA of a microorganism, preferably of the genus Bacteroides microorganism, and more preferably of a Bacteroides reticulotermitis JCM 10512 strain
   (ii-2) A DNA fragment consisting of the nucleotide sequence at positions 607 to 954 in the nucleotide sequence represented by SEQ ID NO: 3, which is obtained by PCR using, as a template, a chromosomal DNA of a microorganism, preferably of the genus Bacteroides microorganism, and more preferably of a Bacteroides reticulotermitis JCM 10512 strain
   (ii-3) A DNA fragment consisting of the nucleotide sequence obtained by codon-optimizing, for expression in Escherichia coli, a nucleotide sequence encoding the amino acid sequence at positions 127 to 214 in the amino acid sequence represented by SEQ ID NO: 6, which is obtained by PCR using, as a template, a chromosomal DNA of a Bacteroides fragilis ATCC 25285 strain

Specific examples of the DNA encoding the protein consisting of the amino acid sequence represented by SEQ ID NO: 2 include a DNA comprising the nucleotide sequence represented by SEQ ID NO: 1.

The DNA encoding the mutant protein of the above [2] can be obtained by, for example, performing error-prone PCR, etc., using a DNA consisting of the nucleotide sequence represented by SEQ ID NO: 7 as a template. The DNA encoding the mutant protein of the above [5] can be obtained by, for example, performing error-prone PCR, etc., using a DNA consisting of the nucleotide sequence represented by SEQ ID NO: 1 as a template.

The DNA in the above [2] and [5] can also be obtained by PCR using a set of PCR primers each having, at the 5' end thereof, a nucleotide sequence designed to insert a target mutation (deletion, substitution, insertion, or addition) [Gene, 77, 51 (1989)].

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced is obtained.

The DNA encoding the homologous protein of the above [3] and the DNA of the above [9] and [10] can be obtained, for example, by a method same as the above method for obtaining the DNA by, for example, searching various gene sequence databases for a nucleotide sequence having an identity of 95% or more, preferably 97% or more, further preferably 98% or more, and most preferably 99% or more with the nucleotide sequence represented by SEQ ID NO: 7, searching various protein sequence databases for an amino acid sequence having an identity of 95% or more, preferably 97% or more, further preferably 98% or more, and most preferably 99% or more with the amino acid sequence represented by SEQ ID NO: 8, and using a probe DNA or a primer DNA that can be designed based on the nucleotide sequence or the amino acid sequence obtained by the search, and a microorganism having the DNA.

The DNA encoding the homologous protein of the above [6] and the DNA of the above [13] and [14] can be obtained, for example, by a method same as the above method for obtaining the DNA by, for example, searching various gene sequence databases for a nucleotide sequence having an identity of 95% or more, preferably 97% or more, further preferably 98% or more, and most preferably 99% or more with the nucleotide sequence represented by SEQ ID NO: 1, searching various protein sequence databases for an amino acid sequence having an identity of 95% or more, preferably 97% or more, further preferably 98% or more, and most preferably 99% or more with the amino acid sequence represented by SEQ ID NO: 2, and using a probe DNA or a primer DNA that can be designed based on the nucleotide sequence or the amino acid sequence obtained by the search, and a microorganism having the DNA.

A nucleotide sequence of the DNA can be determined by using the obtained DNA according to any one of [7] to [14] as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a nucleotide sequence analyzer such as an Applied Biosystems 3500 Genetic Analyzer and an Applied Biosystems 3730 DNA Analyzer (both manufactured by Thermo Fisher Scientific K.K.).

The host cell that can be used for determining the nucleotide sequence of the DNA may be any cell as long as the vector can be introduced and proliferated, and examples thereof include Escherichia coli DH5α, Escherichia coli HST08 Premium, Escherichia coli HST02, Escherichia coli HST04 dam-/dcm-, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990).

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial-length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

The recombinant DNA containing the DNA encoding the protein according to any one of the above [1] to [3] and the protein according to any one of the above [4] to [6] refers to a recombinant DNA obtained by incorporating the DNA into an expression vector which is autonomously replicable in a parent strain or can be incorporated into a chromosome and contains a promoter at a position where the DNA can be transferred.

When the recombinant DNA is a recombinant DNA capable of being incorporated into a chromosome, the recombinant DNA may not contain a promoter.

The microorganism having an increased copy number of the gene as compared with the parent strain, which is obtained by transforming the microorganism of the parent strain with the recombinant DNA containing the DNA encoding the above protein can be obtained by the following method.

Based on the DNA encoding the above protein and obtained by the above method, a DNA fragment of an appropriate length containing a portion encoding the protein is prepared as necessary. A transformant having an improved production rate can be obtained by substituting a base such that a nucleotide sequence of the portion encoding the protein becomes an optimal codon for expression in a host cell.

A recombinant DNA is prepared by inserting the DNA fragment downstream of a promoter of an appropriate expression vector. By transforming the parent strain with the recombinant DNA, a microorganism having an increased copy number of a gene encoding the protein as compared with the parent strain can be obtained.

When a prokaryote such as a bacterium is used as a parent strain, the recombinant DNA is preferably a recombinant DNA composed of a promoter, a ribosome binding sequence, the DNA according to any one of the above [7] to [10] for the protein according to any one of the above [1] to [3], the DNA according to any one of the above [11] to [14] for the protein according to any one of the above [4] to [6], and a transcription termination sequence. A gene for regulating the promoter may be contained.

It is preferable to use a plasmid in which a distance between a Shine-Dalgarno sequence, which is a ribosome binding sequence, and an initiation codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

An expression level of the protein having an α1,2-fucosyltransferase activity or an α1,3-fucosyltransferase activity can be improved by substituting a base such that the nucleotide sequence of the portion encoding the protein having an α1,2-fucosyltransferase activity or an α1,3-fucosyltransferase activity becomes an optimal codon for host expression. Examples of the protein having an α1,2-fucosyltransferase activity include the protein according to any one of the above [1] to [3]. Examples of the protein having an α1,3-fucosyltransferase activity include the protein according to any one of the above [4] to [6]. Information on the frequency of codon use in the parent strain used in the present invention can be obtained through a public database.

The expression vector is not particularly limited as long as it is an appropriate nucleic acid molecule for introducing the target DNA into a host and causing the target DNA to be amplified and expressed. For example, not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW118 and pMW119 (all manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pUAKQE31 (Appl. Environ. Microbiol. 2007, 73: 6378-6385), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK(+), pBluescript II KS(-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p 6378-6385], pPAC31 (WO1998/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from, for example, an Escherichia coli or a phage, such as a uspA promoter, a lac promoter, a PL promoter, a PR promoter, or a PSE promoter can be used. Examples thereof include a promoter which is artificially modified in design, such as a promoter with two trp promoters in series, a tac promoter, a trc promoter, a lacT7 promoter, or a letI promoter.

When a microorganism belonging to the genus Corynebacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

When the above expression vector is used, the promoter may be any promoter as long as it functions in cells of microorganisms belonging to the genus Corynebacterium, and for example, a P54-6 promoter [Appl. Microbiol. Biotechnol., 53, 674-679 (2000)] can be used.

When a yeast strain is used as the parent strain, examples of the expression vector include YEp13 (ATCC 37115), YEp24 (ATCC 37051), YCp50 (ATCC 37419), pHS19, and pHS15.

The promoter in the case of using the above expression vector may be any promoter as long as it functions in cells of the yeast strain, and examples thereof include a PHO5 promoter, a PGK promoter, a GAP promoter, an ADH promoter, a gall promoter, a gal10 promoter, a heat shock polypeptide promoter, an MFα1 promoter, and a CUP1 promoter.

By inserting the DNA fragment according to any one of the above [7] to [10] and the DNA fragment according to any one of the above [11] to [14] downstream of a promoter of an appropriate expression vector, a recombinant DNA to be used in the production method of the present invention can be prepared.

Examples of a method for introducing a recombinant DNA into a parent strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a host cell include a homologous recombination method. Examples of the homologous recombination method include a method using a plasmid for homologous recombination that can be prepared by linking with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6640-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the recombinant DNA is introduced into the parent strain as an autonomously replicable plasmid or incorporated into a chromosome of the parent strain can be confirmed by, for example, a method in which a gene originally contained in a chromosomal DNA of a microorganism cannot be amplified, but the gene introduced through transformation can be amplified by PCR using a primer set to confirm an amplification product. The fact that the transcription amount of the DNA or the production amount of the protein encoded by the DNA is increased can be confirmed by a method of comparing the transcription amount of the gene in the microorganism with that of the parent strain by Northern blotting, or the production amount of the protein in the microorganism with that of the parent strain by Western blotting.

The fact that the microorganism produced by the above method is a microorganism having enhanced activities of the protein according to any one of the above [1] to [3] and the protein according to any one of the above [4] to [6] and having improved productivity of LDFT as compared with a parent strain can be confirmed by appropriately diluting a culture solution after culturing the microorganism, centrifuging the culture solution, and analyzing LDFT contained in a supernatant or in bacterial cells using a carbohydrate analyzer or a high performance liquid chromatograph mass spectrometer to be described later, thereby comparing with that of the parent strain.

The above microorganism has enhanced activities of the protein according to any one of the above [1] to [3] and the protein according to any one of the above [4] to [6] as compared with the parent strain, thereby preventing 2'FL and 3FL from remaining as by-products and improving the productivity of LDFT. Examples of such a microorganism include a FUC/pcBrFucT-HMFT strain in which expression of a cBrFucT gene and an HMFT gene, which will be described in Examples, is enhanced.

In a microorganism having enhanced activities of a cBrFucT protein and an HMFT protein, which is an example of such a microorganism, the activity of the cBrFucT protein to convert 3FL to LDFT with high efficiency is enhanced, and the activity of the HMFT protein to convert 2'FL to LDFT with high efficiency is enhanced, thereby preventing 2'FL and 3FL from remaining as by-products and improving the productivity of LDFT.

### <Method for Producing LDFT>

Examples of a method for producing LDFT of the present invention (hereinafter, also abbreviated as the method of the present invention) include a method for producing a fucose-containing carbohydrate, including preparing the above transformant and producing oligosaccharides in a culture using the transformant.

The above method for culturing the transformant can be performed according to a method generally used for culturing a microorganism. As a culture medium for culturing the transformant, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

As the transformant used in the method for producing LDFT, a microorganism having an ability to produce glucose, lactose, lactose monohydrate, or the like may be used.

In the method for producing LDFT, glucose, lactose, lactose monohydrate, etc. may be added to the culture medium during culture.

When the transformant used in the method for producing LDFT does not have the ability to produce GDP-fucose and/or lactose, GDP-fucose, lactose, or lactose monohydrate may be added to the culture medium.

In the method for producing LDFT, instead of adding glucose, lactose, lactose monohydrate, etc. to the culture medium during culture, glucose, lactose, lactose monohydrate, etc. may be supplied to the transformant of the present invention by culturing a microorganism having an ability to produce glucose, lactose, lactose monohydrate, etc. from sugar simultaneously with the transformant of the present invention.

In the method for producing LDFT, β-galactosidase and WcaJ are preferably absent in the culture medium.

Culture is generally preferably performed under aerobic conditions such as shaking culture, submerged aeration agitation culture, or deep aeration stirring culture. The culture temperature is generally 30°C to 37°C, and the culture time is generally 24 hours to 3 days. The pH of the culture solution during culture is generally maintained at 6.0 to 8.0. The pH is adjusted using an inorganic or organic acid, an alkaline solution, urea, calcium carbonate, ammonia, or the like.

LDFT can be produced by producing LDFT in a culture by the above culture.

Generally, LDFT can be collected from a supernatant after centrifugation of the culture. When LDFT is accumulated in bacterial cells, LDFT can be collected by using an ion exchange resin method or the like from a supernatant obtained by, for example, crushing the bacterial cells with ultrasonic waves or the like and removing the bacterial cells by centrifugation.

A desired fucose-containing carbohydrate can be produced by further adding other saccharides to the LDFT in the culture or to the collected LDFT.

### <Method for Producing LDFT Crystal>

The method for producing an LDFT crystal of the present invention includes the following method:
a method for producing an LDFT crystal from a culture obtained by culturing the microorganism according to the above, the method including the following steps (i) to (iv):
(i) a step of centrifuging the culture to obtain a supernatant from which bacteria cells are removed;
(ii) a step of subjecting the supernatant obtained in step (i) to cation exchange and anion exchange to obtain a solution by removing ions from the supernatant;
(iii) a step of concentrating the solution obtained in the step (ii) with an evaporator; and
(iv) a step of obtaining an LDFT crystal from the solution obtained in step (iii).

Hereinafter, each step will be described.

### (i) A step of centrifuging the culture to obtain a supernatant from which bacteria cells are removed

Step (i) is a step of centrifuging the above culture of the microorganism to obtain a supernatant from which bacteria cells are removed. The culture is preferably heated at 60°C to 80°C for 30 minutes to 120 minutes after adjusting the pH to preferably 3.0 to 4.0 before centrifugation.

Conditions of the centrifugation are not particularly limited, and are generally preferably 0°C to 30°C, and more preferably 0°C to 10°C, preferably 4000 G to 12000 G, or 6000 rpm to 10000 rpm, and preferably 5 minutes to 30 minutes.

### (ii) A step of subjecting the supernatant obtained in step (i) to cation exchange and anion exchange to obtain a solution by removing ions from the supernatant

Step (ii) is a step of subjecting the supernatant which is obtained in step (i) and from which bacteria cells are removed to cation exchange and anion exchange to obtain a solution by removing ions contained in the supernatant. Step (ii) may be performed at room temperature or a lower temperature, and is preferably performed at a lower temperature.

A cation exchange resin is not particularly limited, various ion exchange resins can be used as appropriate, and a strongly acidic cation exchange resin is preferred. The strongly acidic cation exchange resin has, for example, a sulfonic acid group in a crosslinked styrene skeleton. More specifically, examples thereof include resins such as DIAION UBK 550 (H⁺ type) (manufactured by Mitsubishi Chemical Group Corporation) and Marathon C (H⁺ type) (manufactured by Dow Chemical).

An anion exchange resin is not particularly limited, various ion exchange resins can be used as appropriate, and a weakly basic anion exchange resin is preferred. The weakly basic anion exchange resin has, for example, a primary to tertiary amine group in a crosslinked styrene skeleton, or a carboxylic acid in a crosslinked acrylic skeleton. More specifically, examples thereof include resins such as A111S (OH⁻ type) (manufactured by Prolite), A845S (OH⁻ type) (manufactured by Prolite), and WA30 (OH⁻ type) (manufactured by Mitsubishi Chemical Group Corporation).

In the cation exchange and the anion exchange, one type of cation exchange resin and one type of anion exchange resin may be used. If necessary, a plurality of cation exchange resins and a plurality of anion exchange resins may be used in combination. After the cation exchange, the anion exchange may be performed. Alternatively, the cation exchange may be performed after the anion exchange.

The pH in the cation exchange is not particularly limited, and is generally preferably 0 to 10, and more preferably 0 to 5. The pH in the anion exchange is not particularly limited, and is generally preferably 4 to 14, and more preferably 9 to 14.

A column filled with the cation exchange resin may be any column as long as it is a column used for purifying a chemical substance. When the column is filled with the ion exchange resin, a ratio of a resin layer height/a column inner diameter is preferably selected to be large, and the ratio of a resin layer height/a column inner diameter is more preferably selected to be 3 or more.

A column filled with the anion exchange resin may be any column as long as it is a column used for purifying a chemical substance. When the column is filled with the ion exchange resin, a ratio of a resin layer height/a column inner diameter is preferably selected to be large, and the ratio of a resin layer height/a column inner diameter is more preferably selected to be 3 or more.

In order to pass the supernatant which is obtained in step (i) and from which bacteria cells are removed through the column filled with the cation exchange resin, for example, the solution may be passed through the column filled with the ion exchange resin from an upper portion of the column, that is, a so-called column bed upper layer, or may be passed through the column from a lower portion of the column, that is, a so-called column bed lower layer. As a column passing rate, a space velocity is preferably 5 [1/hour] or less, and more preferably 3 [1/hour] or less.

In order to pass the supernatant which is obtained in step (i) and from which bacteria cells are removed through the column filled with the anion exchange resin, for example, the solution may be passed through the column filled with the ion exchange resin from an upper portion of the column, that is, a so-called column bed upper layer, or may be passed through the column from a lower portion of the column, that is, a so-called column bed lower layer. As a column passing rate, a space velocity is preferably 5 [1/hour] or less, and more preferably 3 [1/hour] or less.

A fraction obtained after the cation exchange and the anion exchange may be filtered. For filtration, an MF membrane (HVLP 06225, manufactured by Merck Co., Ltd.), an MF membrane module (PSP-003, manufactured by Asahi Kasei Corporation), a UF membrane (VVLP 06225, manufactured by Merck Co., Ltd.), a UF membrane module (SIP-0013, manufactured by Asahi Kasei Corporation), or a filtration membrane similar to those described above can be used.

### (iii) A step of concentrating the solution obtained in step (ii) with an evaporator

Step (iii) is a step of concentrating the solution obtained in step (ii) with an evaporator to obtain a solution containing LDFT with high purity.

In order to obtain the solution containing LDFT with high purity, a concentration step is performed. The concentration step is generally performed under vacuum at a pressure in a range of, for example, 0 mb to 2 mb, and preferably 0 mb to 1.2 mb. The vacuum makes it possible to lower the temperature required for evaporation and to reduce the duration of the concentration step. This can be performed at a temperature in a range of 5°C to 60°C, preferably 5°C to 50°C, and further preferably 5°C to 40°C.

The concentration step can be performed using a one-step evaporator, and a multi-stage evaporator, for example, a two-stage evaporator. The concentration step can be performed continuously.

The purity of LDFT in the solution containing LDFT with high purity is preferably 50% or more, and further preferably 80% or more.

### (iv) A step of obtaining an LDFT crystal from the solution obtained in step (iii)

Step (iv) is a step of obtaining an LDFT crystal from the solution containing LDFT that is obtained in step (iii).

An LDFT crystal is added as a seed crystal to the solution containing LDFT. As the LDFT crystal to be added as a seed crystal, a crystal obtained by the method of the present invention can be used. The seed crystal may be added before the crystal precipitation step or during the crystal precipitation step, as long as the LDFT crystal can be precipitated in the solution. The seed crystal is added such that a concentration in the solution is generally 0.1 wt% to 10 wt%, and preferably 0.1 wt% to 2.0 wt%.

Examples of the method for precipitating an LDFT crystal in the solution include a method for dropping a poor solvent to the solution. The poor solvent refers to a solvent in which a chemical substance has a small solubility. A mixture of the poor solvent and the solvent may be used. The poor solvent may be preferably ethanol, methanol, n-propanol, isopropyl alcohol, or acetone. When the solution is mixed, the solubility of the substance in the solvent mixture decreases, and the substance is precipitated.

After the LDFT crystal is precipitated as described above and before the step of collecting the precipitated crystal, the precipitated crystal may be further aged for generally 0.5 hours to 12 hours, preferably 2 hours to 12 hours, and most preferably 2 hours to 6 hours.

The term "aged" refers to temporarily stopping the step of precipitating the LDFT crystal and growing the crystal. After the crystal is aged, the step of precipitating the LDFT crystal may be resumed.

The method for collecting an LDFT crystal is not particularly limited, and examples thereof include filtration, pressure filtration, suction filtration, and centrifugation. Further, in order to reduce adhesion of a mother liquor to the crystal and improve the quality of the crystal, the crystal can be appropriately washed after the crystal is collected. A solution used for crystal washing is not particularly limited, and a solution obtained by mixing one kind or a plurality of kinds selected from water, methanol, ethanol, acetone, n-propanol, and isopropyl alcohol at any ratio can be used.

The crystal of the present invention can be obtained by drying the wet crystal thus obtained. That is, the method for producing a crystal of the present invention may further include a step of drying the LDFT crystal.

The drying conditions may be any method as long as the form of the LDFT crystal can be maintained, such as reduced pressure drying, vacuum drying, fluidized bed drying, or ventilation drying.

The drying temperature may be any range as long as it can remove attached moisture or solution, and is preferably 80°C or lower, more preferably 60°C or lower, and further preferably 10°C or lower.

By the above method, a high-purity LDFT crystal can be obtained. The crystal of the present invention is preferably an LDFT•7.2 hydrates crystal.

The fact that the crystal of the present invention is a 7.2 hydrate crystal can be confirmed by single crystal X-ray analysis to be described later. The purity of the obtained LDFT crystal is generally 80% or more, preferably 90%, more preferably 97% or more, 98% or more, further preferably 99% or more, and most preferably 99.5% or more in this order.

Examples of the method for analyzing a structure of the obtained LDFT crystal include powder X-ray diffraction and single crystal X-ray diffraction using CuKα as an X-ray source.

In step (iv), when the LDFT crystal is obtained by dropping a poor solvent into the solution obtained in step (iii), in the case of using, as the poor solvent, for example, ethanol, methanol, n-propanol, isopropyl alcohol, or acetone other than water, the LDFT crystal obtained by the above method is preferably an LDFT crystal having a peak at a diffraction angle (2θ) described in the following (i) in powder X-ray diffraction using CuKα as an X-ray source, and more preferably an LDFT crystal having a peak at a diffraction angle (2θ) described in the following (ii) in addition to the diffraction angle (2θ) described in the following (i).
(i) 11.1, 15.8, 18.4, 18.7, 21.7
(ii) 22.8, 16.6, 23.0, 22.6, 20.0

### [Analysis Example]

### (1) Analysis and Quantification of LDFT, 2'FL, 3FL, or Lactose

In Examples, analysis and quantification of LDFT, 2'FL, 3FL, or lactose were performed according to the procedures shown below.

A culture solution containing microorganisms after culture was centrifuged, and a supernatant was collected. LDFT, 2'FL, 3FL, or lactose contained in the supernatant was analyzed by a carbohydrate analyzer ICS-5000 (manufactured by Thermo Fisher Scientific K.K.).

### [Analysis Conditions]

Column: CarboPAC PA1
Column temperature: 25°C

### Mobile phase:

(mobile phase A) water
(mobile phase B) 500 mmol/L sodium hydroxide

(mobile phase C) 300 mmol/L sodium acetate

### Mixing ratio of mobile phase A, mobile phase B, and mobile phase C:

(0 minutes to 10 minutes) 80:20:0
(10 minutes to 15 minutes) gradient from 80:20:0 to 70:20:10
(15 minutes to 17 minutes) gradient from 70:20: 10 to 0:20:80
(17 minutes to 25 minutes) 0:20:80
(25 minutes to 35 minutes) 80:20:0
Flow rate: 1.0 mL/min
Detector: pulsed amperometric detector

### (2) Single Crystal X-ray Structure Analysis

Single crystal X-ray structure analysis was performed using a single crystal X-ray diffractometer Synergy-R (manufactured by Rigaku Corporation), and the measurement was performed according to an instruction manual.

### (3) Powder X-ray Diffraction

Powder X-ray diffraction was performed using a powder X-ray diffractometer (XRD) Ultima IV (manufactured by Rigaku Corporation), and the measurement was performed according to an instruction manual.

### EXAMPLE

Examples of the present invention are shown below, but the present invention is not limited to these Examples.

### [Example 1] Acquisition of α1,2-fucosyltransferase Useful for Producing LDFT

Using an activity of transferring a fucose to lactose or 3FL as an indicator, α1,2-fucosyltransferase useful for producing LDFT was screened.

### (1) Construction of Host Strain for Evaluation

### <Acquisition of DNA Fragment to be Used as Marker for Gene Deletion>

PCR was performed using a DNA consisting of the nucleotide sequences represented by SEQ ID NOs: 15 and 16 as a primer set and pCatSac (Appl Environ Microbiol (2013) 79, 3033-3039) as a template to obtain a cat-sacB fragment containing a chloramphenicol-resistant cat gene and a sucrose sensitive sacB gene.

### <Construction of Escherichia Coli Lacking β-galactosidase Activity, Lactose Permease Activity, and Cholanic Acid Synthesis Activity>

Escherichia coli deficient in DNA encoding β-galactosidase (hereinafter, referred to as lacZ gene), DNA encoding lactose permease (hereinafter, referred to as lacY gene), and DNA encoding a cholanic acid production-related protein (hereinafter, referred to as wcaJ, wzxC, wcaK, wcaL, or wcaM gene) was constructed by the following method. Note that the lacZ and lacY (hereinafter, referred to as lacZY), and wcaJ, wzxC, wcaK, wcaL, and wcaM (hereinafter, referred to as wcaJ-wzxC-wcaKLM) each form an operon on the Escherichia coli genome.

PCR was performed using, as a template, a genomic DNA of the Escherichia coli W3110 strain prepared by an ordinary method and using, as a primer set, a DNA consisting of the nucleotide sequences represented by "Primer set" in Table 1 to amplify each DNA fragment.

**[Table 1]**

| Primer set (SEQ ID NOs:) | Amplified DNA fragment | Remarks |
|---|---|---|
| 17 and 18 | lacZ upstream 1 | Sequences in nucleotide sequences represented by SEQ ID NOs: 15 and 17 at 5' ends are complementary |
| 19 and 20 | lacY downstream 1 | Sequences in nucleotide sequences represented by SEQ ID NOs: 16 and 19 at 5' ends are complementary |
| 18 and 21 | lacZ upstream 2 | Sequences in nucleotide sequences represented by SEQ ID NOs: 21 and 22 at 5' ends are complementary |
| 20 and 22 | lacY downstream 2 | |

The lacZ upstream 1 and lacZ upstream 2 include a region from an initiation codon of the lacZ gene to about 1000 bp upstream of the initiation codon. The lacY downstream 1 and lacY downstream 2 contain a region from about 50 bp to about 1000 bp downstream of a stop codon of the lacY gene.

PCR was performed using, as a template, a mixture of the lacZ upstream 1, the lacY downstream 1, and the cat-sacB fragment at an equimolar ratio and using, as a primer set, a DNA consisting of the nucleotide sequences represented by SEQ ID NOs: 18 and 20 to obtain a DNA (hereinafter, referred to as lacZY::cat-sacB) fragment consisting of a sequence with the cat-sacB fragment inserted in a region around the lacZ and lacY genes.

PCR was performed using, as a template, a mixture of the lacZ upstream 2 and the lacY downstream 2 at an equimolar ratio and using, as a primer set, a DNA consisting of the nucleotide sequences represented by SEQ ID NOs: 18 and 20 to obtain a DNA (hereinafter, referred to as ΔlacZY) fragment consisting of a sequence with the lacZ upstream and the lacY downstream directly linked to each other without lacZY.

The lacZY::cat-sacB fragment was introduced into a W3110 strain carrying a plasmid pKD46 comprising a gene encoding λ recombinase [Datsenko, K. A., Warner, B. L. L., Proc. Natl. Acad.Sci, USA, Vol. 97, 6640-6645, (2000)] by an electroporation method to obtain a transformant (a transformant with the lacZY gene substituted with lacZY::cat-sacB) exhibiting chloramphenicol resistance and sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant without pKD46) exhibiting ampicillin sensitivity was further obtained. The transformant was named W3110ΔlacZY.

Similarly, PCR was performed using, as a template, the genomic DNA of the W3110 strain and using, as a primer set, a DNA consisting of the nucleotide sequences represented by "Primer set" in Table 2 to obtain each amplified DNA fragment.

**[Table 2]**

| Primer set (SEQ ID NOs:) | Amplified DNA fragment | Remarks |
|---|---|---|
| 23 and 24 | wcaJ upstream 1 | Sequences in nucleotide sequences represented by SEQ ID NOs: 15 and 23 at 5' ends are complementary |
| 25 and 26 | wcaM downstream 1 | Sequences in nucleotide sequences represented by SEQ ID NOs: 16 and 25 at 5' ends are complementary |
| 24 and 27 | wcaJ upstream 2 | Sequences in nucleotide sequences represented by SEQ ID NOs: 27 and 28 at 5' ends are complementary |
| 26 and 28 | wcaM downstream 2 | |

The wcaJ upstream 1 and the wcaJ upstream 2 contain a region from an initiation codon of the wcaJ gene to about 1000 bp upstream of the initiation codon. The wcaM downstream 1 and the wcaM downstream 2 contain a region from a stop codon of the wcaM gene to about 1000 bp downstream of the stop codon.

PCR was performed using, as a template, a mixture of the wcaJ upstream 1, the wcaM downstream 1, and the cat-sacB fragment at an equimolar ratio and using, as a primer set, a DNA consisting of the nucleotide sequences represented by SEQ ID NOs: 24 and 26 to obtain a DNA (hereinafter, referred to as wcaJ-wzxC-wcaKLM::cat-sacB) fragment consisting of a sequence with the cat-sacB fragment inserted in a region around a wcaJ-wzxC-wcaKLM operon.

PCR was performed using, as a template, a mixture of the wcaJ upstream 2 and the wcaM downstream 2 at an equimolar ratio and using, as a primer set, a DNA consisting of the nucleotide sequences represented by SEQ ID NOs: 24 and 26 to obtain a DNA (hereinafter, referred to as ΔwcaJ-wzxC-wcaKLM) fragment consisting of a sequence with the wcaJ upstream and the wcaM downstream directly linked to each other without wcaJ-wzxC-wcaKLM.

The wcaJ-wzxC-wcaKLM::cat-sacB fragment was introduced into the W3110 ΔlacZY strain constructed as described above by the electroporation method to obtain a transformant (a transformant with wcaJ-wzxC-wcaKLM substituted with wcaJ-wzxC-wcaKLM::cat-sacB) exhibiting chloramphenicol resistant and sucrose sensitivity.

The ΔwcaJ-wzxC-wcaKLM fragment was introduced into the transformant by the electroporation method to obtain a transformant (a transformant with wcaJ-wzxC-wcaKLM::cat-sacB substituted with ΔwcaJ-wzxC-wcaKLM) exhibiting chloramphenicol sensitivity and sucrose resistance. Further, a transformant (a transformant without pKD46) exhibiting ampicillin sensitivity was obtained. The transformant was named FUC strain.

### (2) Construction of Microorganism having α1,2-Fucosyltransferase Activity

Escherichia coli having a gene encoding various types of α1,2-fucosyltransferases arranged under the uspA promoter and having a plasmid for expressing the gene was constructed by the following method.

### <Construction of expression vector>

PCR was performed using, as a template, the genomic DNA of the W3110 strain prepared by an ordinary method and using, as a primer set, a DNA consisting of the nucleotide sequences represented by "Primer set" in Table 3 to obtain each amplified DNA fragment.

**[Table 3]**

| Primer set (SEQ ID NOs:) | Amplified DNA fragment | Remarks |
|---|---|---|
| 35 and 36 | rcsA | Sequences in nucleotide sequences represented by SEQ ID NOs: 36 and 37 at 5' ends are complementary |
| 37 and 38 | lacY | |

PCR was performed using, as a template, a mixture of the rcsA fragment and the lacY fragment at an equimolar ratio and using, as a primer set, a DNA consisting of the nucleotide sequences represented by SEQ ID NOs: 35 and 38 to obtain a DNA (hereinafter, referred to as rcsA-lacY) fragment with the two fragments linked.

PCR was performed using, as a primer set, a DNA consisting of the nucleotide sequences represented by SEQ ID NOs: 33 and 34 and using, as a template, a plasmid pUAKQE 31 (Appl. Environ. Microbiol. 2007, 73: 6378-6385) to obtain a vector fragment of about 4.7 kb.

In this case, the nucleotide sequences represented by SEQ ID NOs: 33 and 35 and SEQ ID NOs: 34 and 38 each contain a complementary sequence at the 5' end.

The rcsA-lacY fragment and the vector fragment obtained above were ligated using an In-Fusion HD Cloning Kit (manufactured by Takara Bio Inc.) to obtain an expression vector pUAKQE-rcsA-lacY.

### <Construction of Plasmid for Expressing α1,2-Fucosyltransferase>

PCR was performed using, as a primer set, a DNA consisting of the nucleotide sequences shown in "Primer set" in Table 4 and using, as a template, a DNA represented by "Template" in Table 4 to obtain each amplified DNA fragment.

**[Table 4]**

| Primer set (SEQ ID NOs:) | Template | Amplified DNA fragment |
|---|---|---|
| 40 and 41 | Genomic DNA of Helicobacter mustelae ATCC 43772 | HMFT (SEQ ID NO: 7) |
| 42 and 43 | DNA represented by SEQ ID NO: 9 | FutC (SEQ ID NO: 9) |
| 44 and 45 | DNA represented by SEQ ID NO: 11 | Te2FT (SEQ ID NO: 11) |

The genomic DNA of the Helicobacter mustelae ATCC 43772 strain was prepared by an ordinary method.

The DNA represented by SEQ ID NO: 9 was a nucleotide sequence of a gene encoding α1,2-fucosyltransferase FutC derived from a Helicobacter pylori 26695 strain and represented by SEQ ID NO: 10, and was prepared by artificial synthesis.

The DNA represented by SEQ ID NO: 11 was a DNA in which a nucleotide sequence of the gene encoding α1,2-fucosyltransferase Te2FT derived from the Thermosynechococcus elongatus BP-1 strain and represented by SEQ ID NO: 12 was codon-optimized for expression in Escherichia coli, and was prepared by artificial synthesis.

PCR was performed using the expression vector pUAKQE-rcsA-lacY constructed by the above-described method as a template and a DNA consisting of the nucleotide sequences represented by SEQ ID NOs: 33 and 39 as a primer set to obtain a vector fragment of about 6.7 kb. The nucleotide sequences represented by SEQ ID NOs: 40, 42, 44 and 33, and SEQ ID NOs: 41, 43, 45, and 39 each contain a complementary sequence at the 5' end.

The various amplified DNA fragments and vector fragments obtained above were linked using an In-Fusion HD Cloning Kit (manufactured by Takara Bio Inc.) to construct plasmids expressing various types of α1,2-fucosyltransferase, pHMFT, pFutC, and pTe2FT.

### <Construction of Escherichia Coli having Plasmid for Expressing α1,2-Fucosyltransferase>

The FUC strain constructed in Example 1(1) was transformed with the above-obtained α1,2-fucosyltransferase expression plasmids, pHMFT, pFutC, and pTe2FT and pUAKQE-rcsA-lacY as a vector control to construct Escherichia Coli strains having various plasmids, which were named FUC/pHMFT strain, FUC/pFutC strain, FUC/pTe2FT strain, and FUC/Ctrl strain, respectively.

### (3) Evaluation of Various Types of α1,2-Fucosyltransferase using Productivity of 2'FL and LDFT as Indicator

The productivity of 2'FL and LDFT was evaluated for the FUC/pHMFT strain, FUC/pFutC strain, FUC/pTe2FT strain, and FUC/Ctrl strain, which were obtained in the above (2).

Each strain was cultured on an LB plate containing 100 mg/L kanamycin at 37°C for 16 hours, inoculated into a test tube containing 2 mL of LB culture medium containing 100 mg/L kanamycin, and cultured with shaking at 30°C for 16 hours. Thereafter, 0.2 mL of each obtained culture solution was inoculated into a large-sized test tube containing 4 mL of a production culture medium [glucose 30 g/L, lactose monohydrate or 3FL 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (adjusted to pH 7.2 by aqueous sodium hydroxide, except for glucose, lactose monohydrate, 3FL, and magnesium sulfate heptahydrate, and then autoclaved) (aqueous solutions containing glucose, lactose monohydrate, 3FL, and magnesium sulfate heptahydrate were separately prepared, autoclaved, cooled, and then mixed)] containing 100 mg/L of kanamycin, and cultured with shaking at 30°C for 29 hours.

At this time, when evaluating the productivity of 2'FL, a production culture medium containing lactose monohydrate was used, and when evaluating the productivity of LDFT, a production culture medium containing 3FL was used. After completion of the culture, the culture solution was centrifuged and then appropriately diluted, and 2'FL or LDFT contained in the supernatant was analyzed using a carbohydrate analyzer ICS-5000. The results are shown in Table 5.

**[Table 5]**

| Strain | 2'FL [g/L] | LDFT [g/L] |
|---|---|---|
| FUC/pHMFT | 8.25 | 0.22 |
| FUC/pFutC | 2.62 | 0.69 |
| FUC/pTe2FT | 1.28 | N.D. |
| FUC/Ctrl | N.D. | N.D. |

First, it was confirmed that under lactose-supplemented conditions, all α1,2-fucosyltransferase-expressing strains had an ability to produce 2'FL. Among them, the FUC/pHMFT strain had the highest 2'FL productivity, indicating that HMFT is a useful α1,2 fucosyltransferase to supply an intermediate 2'FL in the LDFT production process from lactose.

Next, under 3FL-supplemented conditions, the production of LDFT was not confirmed in the FUC/pTe2FT strain, but it was found that the FUC/pHMFT strain and the FUC/pFucC strain were able to produce LDFT, albeit in small amounts.

### [Example 2] Acquisition of α1,3-Fucosyltransferase Useful for Producing LDFT

Using an activity of transferring fucose to lactose or 2'FL as an indicator, α1,3-fucosyltransferase useful for producing LDFT was screened.

### (1) Construction of Host Strain for Evaluation

In order to evaluate 3FL productivity, Escherichia coli having a plasmid for expressing a gene encoding a 3FL transporter (hereinafter, referred to as MdfA) belonging to the drug:H⁺ antiporter-1 family derived from the W3110 strain and consisting of the amino acid sequence represented by SEQ ID NO: 14 was constructed by the following method.

PCR was performed using a DNA consisting of the nucleotide sequences represented by SEQ ID NOs: 31 and 32 as a primer set and the genomic DNA of the W3110 strain prepared by an ordinary method as a template to obtain an mdfA fragment. PCR was performed using a DNA consisting of the nucleotide sequences represented by SEQ ID NOs: 29 and 30 as a primer set and using a plasmid pMW118 (manufactured by NIPPON GENE CO., LTD.) as a template to obtain a vector fragment of about 4.1 kb. In this case, the nucleotide sequences represented by SEQ ID NOs: 29 and 31 and SEQ ID NOs: 30 and 32 each contain a complementary sequence at the 5' end.

The mdfA fragment and vector fragment obtained above were ligated using an In-Fusion HD Cloning Kit (manufactured by Takara Bio Inc.) to obtain an MdfA expression plasmid pMW118_mdfA.

The FUC strain constructed in Example 1(1) was transformed with the expression plasmid pMW118_mdfA to construct Escherichia coli having pMW118_mdfA, which was named FUCM strain.

### (2) Construction of Microorganism having α1,3-Fucosyltransferase Activity

Escherichia coli having a gene encoding various types of α1,2-fucosyltransferases disposed under the uspA promoter and having a plasmid for expressing the gene was constructed by the following method.

### <Construction of Plasmid for Expressing α1,3-Fucosyltransferase>

PCR was performed using, as a primer set, a DNA consisting of the nucleotide sequences shown in "Primer set" in Table 6 and using, as a template, a DNA represented by "Template" in Table 6 to obtain each amplified DNA fragment.

**[Table 6]**

| Primer set (SEQ ID NOs:) | Template | Amplified DNA fragment |
|---|---|---|
| 46 and 47 | Genomic DNA of Bacteroides fragilis ATCC 25285 | BfFucT (SEQ ID NO: 5) |
| 48 and 49 | DNA represented by SEQ ID NO: 3 | HpFutA (SEQ ID NO: 3) |

A genomic DNA of Bacteroides fragilis ATCC 25285 strain was prepared by an ordinary method.

The DNA represented by SEQ ID NO: 3 was a nucleotide sequence of a gene encoding α1,3-fucosyltransferase HpFutA derived from a Helicobacter pylori 26695 strain and represented by SEQ ID NO: 4, and was prepared by artificial synthesis.

Subsequently, a gene encoding modified type α1,3-fucosyltransferase consisting of the amino acid sequence represented by SEQ ID NO: 2 and cBrFucT was prepared by the following method.

PCR was performed using, as a primer set, a DNA consisting of the nucleotide sequences shown in "Primer set" in Table 7 and using, as a template, a DNA represented by "Template" in Table 7 to obtain each amplified DNA fragment.

**[Table 7]**

| Primer set (SEQ ID NOs:) | Template | Amplified DNA fragment |
|---|---|---|
| 50 and 51 | Genomic DNA of Bacteroides reticulotermitis JCM10512 strain | cBrFucT upstream |
| 52 and 53 | DNA consisting of nucleotide sequence represented by SEQ ID NO: 64 | CBrFucT midstream |
| 54 and 55 | Genomic DNA of Bacteroides reticulotermitis JCM10512 strain | cBrFucT downstream |

A genomic DNA of the Bacteroides reticulotermitis JCM 10512 strain was prepared by an ordinary method. A DNA consisting of the nucleotide sequence represented by SEQ ID NO: 64 is a DNA obtained by codon-optimizing, for expression in Escherichia coli, a partial sequence of a gene encoding Bacteroides fragilis-derived α1,3-fucosyltransferase (hereinafter, referred to as BfFucT). The nucleotide sequences represented by SEQ ID NOs: 51 and 52 and SEQ ID NOs: 53 and 54 each contain a complementary sequence at the 5' end.

PCR was performed using, as a template, a mixture of three fragments of the cBrFucT upstream, the cBrFucT midstream, and the cBrFucT downstream at an equimolar ratio and using, as a primer set, a DNA consisting of the nucleotide sequences represented by SEQ ID NOs: 50 and 55 to obtain a DNA fragment with the three fragments linked (hereinafter, referred to as cBrFucT fragment).

PCR was performed using the expression vector pUAKQE-rcsA-lacY constructed by the above-described method as a template and a DNA consisting of the nucleotide sequences represented by SEQ ID NOs: 33 and 39 as a primer set to obtain a vector fragment of about 6.7 kb. The nucleotide sequences represented by SEQ ID NOs: 46, 48, 50, and 33, and SEQ ID NOs: 47, 49, 55, and 39 each contain a complementary sequence at the 5' end.

The various amplified DNA fragments BfFucT, HpFutA, or cBrFucT obtained above were linked to a vector fragment using an In-Fusion HD Cloning Kit (manufactured by Takara Bio Inc.) to construct plasmids expressing various types of α1,3-fucosyltransferase, pBfFucT, pHpFutA, and pcBrFucT.

### <Construction of Escherichia Coli having Plasmid for Expressing α1,3-Fucosyltransferase>

The FUCM strain constructed in the above (1) was transformed with the α1,3-fucosyltransferase expression plasmids, pBfFucT, pHpFutA, and pcBrFucT obtained above, and pUAKQE-rcsA-lacY as a vector control to construct Escherichia Coli strains having various plasmids, which were named FUCM/pBfFucT, FUCM/pHpFutA strain, FUCM/pcBrFucT strain, and FUCM/Ctrl strain, respectively.

### (3) Evaluation of Various Types of α1,3-Fucosyltransferase using Productivity of 3FL and LDFT as Indicator

The productivity of 3FL and LDFT was evaluated for the FUCM/pBfFucT, FUCM/pHpFutA, FUCM/pcBrFucT and FUCM/Ctrl strains obtained in the above (2).

Each strain was cultured on an LB plate containing 100 mg/L kanamycin and 100 mg/L ampicillin at 37°C for 16 hours, then inoculated into a test tube containing 2 mL of LB culture medium containing 100 mg/L kanamycin and 100 mg/L ampicillin, and cultured with shaking at 30°C for 16 hours.

Thereafter, 0.2 mL of each obtained culture solution was inoculated into a large-sized test tube containing 4 mL of a production culture medium [glucose 30 g/L, lactose monohydrate or 2'FL 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (adjusted to pH 7.2 by aqueous sodium hydroxide, except for glucose, lactose monohydrate, 2FL, and magnesium sulfate heptahydrate, and then autoclaved) (aqueous solutions containing glucose, lactose monohydrate, 2FL, and magnesium sulfate heptahydrate were separately prepared, autoclaved, cooled, and then mixed)] containing 100 mg/L of kanamycin and 100 mg/L ampicillin, and cultured with shaking at 30°C for 29 hours. For the FUCM strain-derived producer bacteria, IPTG was added 6 hours after the start of culture to a final concentration of 1 mM.

At this time, when evaluating the productivity of 3FL, a production culture medium containing lactose monohydrate was used, and when evaluating the productivity of LDFT, a production culture medium containing 2'FL was used.

After completion of the culture, the culture solution was centrifuged and then appropriately diluted, and the 3FL or LDFT contained in the supernatant was analyzed using a carbohydrate analyzer ICS-5000. The results are shown in Tables 8 and 9.

**[Table 8]**

| Strain | 3FL [g/L] | LDFT [g/L] |
|---|---|---|
| FUCM/pBfFucT | 0.88 | 0.98 |
| FUCM/pHpFutA | 0.33 | 0.32 |
| FUCM/Ctrl | N.D. | N.D. |

**[Table 9]**

| Strain | 3FL [g/L] |
|---|---|
| FUCM/pcBrFucT | 2.14 |
| FUCM/pBfFucT | 0.92 |
| FUCM/Ctrl | N.D. |

First, it was confirmed that under lactose-supplemented conditions, all α1,3-fucosyltransferase-expressing strains had the ability to produce 3FL. Among them, the FUCM/pcBrFucT strain had the highest 3FL productivity, indicating that cBrFucT is a useful α1,3 fucosyltransferase to supply an intermediate 3FL in the LDFT production process from lactose.

Next, under 2'FL-supplemented conditions, the FUCM/pBfFucT strain and the FUCM/pHpFucA strain were confirmed to produce LDFT in amounts roughly equivalent to that of 3FL. This result suggested that BfFucT and HpFucA may be equally capable of utilizing lactose and 2'FL as substrates.

### [Example 3] Construction of Microorganism Used for Producing LDFT

Escherichia Coli used in Examples 1 and 2, which has a plasmid co-expressing the gene encoding α1,3-fucosyltransferase and the gene encoding α1,2-fucosyltransferase, was constructed by the following method.

PCR was performed using, as a template, the DNA constructed in Example 1 or 2, as shown in "Template" in Table 10 and using, as a primer set, a DNA consisting of the nucleotide sequences represented by "Primer set" in Table 10 to amplify each DNA fragment.

**[Table 10]**

| Primer set (SEQ ID NOs:) | Template | Amplified DNA fragment | Produced plasmid |
|---|---|---|---|
| 50 and 56 | pcBrFucT | cBrFucT (SEQ ID NO: 1) | pcBrFucT-HMFT |
| 57 and 41 | pHMFT | HMFT (SEQ ID NO: 7) | |
| 46 and 58 | pBfFucT | BfFucT (SEQ ID NO: 5) | pBfFucT-HMFT |
| 57 and 41 | pHMFT | HMFT (SEQ ID NO: 7) | |
| 48 and 59 | pHpFutA | HpFutA (SEQ ID NO: 3) | pHpFutA-HMFT |
| 57 and 41 | pHMFT | HMFT (SEQ ID NO: 7) | |
| 50 and 60 | pcBrFucT | cBrFucT (SEQ ID NO: 1) | pcBrFucT-FutC |
| 61 and 43 | pFutC | FutC (SEQ ID NO: 9) | |
| 50 and 62 | pcBrFucT | cBrFucT (SEQ ID NO: 1) | pcBrFucT-Te2FT |
| 63 and 45 | pTe2FT | Te2FT (SEQ ID NO: 11) | |

The nucleotide sequences represented by SEQ ID NOs: 56 and 57, SEQ ID NOs: 58 and 57, SEQ ID NOs: 59 and 57, SEQ ID NOs: 60 and 61, and SEQ ID NOs: 62 and 63 each contain a complementary sequence at the 5' end.

PCR was performed using, as a template, a mixture of each amplified DNA fragment at an equimolar ratio in the combination of cBrFucT and HMFT, BfFucT and HMFT, HpFutA and HFT, cBrFucT and FutC, or cBrFucT and Te2FT, and using, as a primer set, a DNA consisting of the nucleotide sequences represented by SEQ ID NOs: 50 and 41, 46 and 41, 48 and 41, 50 and 43, or 50 and 45 to obtain DNA fragments in which an α1,3-fucosyltransferase fragment and an α1,2-fucosyltransferase fragment were linked, which were named cBrFucT-HMFT, BfFucT-HMFT, HpFutA-HMFT, cBrFucT-FutC, and cBrFucT-Te2FT.

PCR was performed using the expression vector pUAKQE-rcsA-lacY constructed in Example 1(2) as a template and a DNA consisting of the nucleotide sequences represented by SEQ ID NOs: 33 and 39 as a primer set to obtain a vector fragment of about 6.7 kb. The nucleotide sequence represented by SEQ ID NOs: 50, 46, 48, and 33, and SEQ ID NOs: 41, 43, 45, and 39 each contain a complementary sequence at the 5' end.

The DNA fragments obtained by ligation as described above and the vector fragments were ligated using an In-Fusion HD Cloning Kit (manufactured by Takara Bio Inc.) to obtain plasmids co-expressing α1,3-fucosyltransferase and α1,2-fucosyltransferase, pcBrFucT-HMFT, pBfFucT-HMFT, pHpFutA-HMFT, pcBrFucT-FutC, and pcBrFucT-Te2FT.

The FUC strain constructed in Example 1(1) was transformed with the constructed plasmids pcBrFucT-HMFT, pBfFucT-HMFT, pHpFutA-HMFT, pcBrFucT-FutC, and pcBrFucT-Te2FT, and pUAKQE-rcsA-lacY constructed in Example 1(2) to obtain FUC/pcBrFucT-HMFT strain, FUC/pBfFucT-HMFT strain, FUC/pHpFutA-HMFT strain, FUC/pcBrFucT-FutC strain, FUC/pcBrFucT-Te2FT strain, and FUC/Ctrl strain, respectively.

### [Example 4] Production of LDFT

The productivity of LDFT from lactose was evaluated for the FUC/pcBrFucT-HMFT strain, FUC/pBfFucT-HMFT strain, FUC/pHpFutA-HMFT strain, FUC/pcBrFucT-FutC strain, FUC/pcBrFucT-Te2FT strain, and FUC/Ctrl strain, which were obtained in Example 3.

Each strain was cultured on an LB plate containing 100 mg/L kanamycin at 37°C for 16 hours, inoculated into a test tube containing 2 mL of LB culture medium containing 100 mg/L kanamycin, and cultured with shaking at 30°C for 15 hours.

Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (adjusted to pH 7.2 by aqueous sodium hydroxide, except for glucose, lactose monohydrate, and magnesium sulfate heptahydrate, and then autoclaved) (aqueous solutions containing glucose, lactose monohydrate, and magnesium sulfate heptahydrate were separately prepared, autoclaved, cooled, and then mixed)] containing 100 mg/L of kanamycin, and cultured with shaking at 30°C for 29 hours.

After completion of the culture, the culture solution was centrifuged and then appropriately diluted, and the LDFT, 2'FL, or 3FL contained in the supernatant was analyzed using a carbohydrate analyzer ICS-5000. The results are shown in Table 11.

**[Table 11]**

| Strain | LDFT [g/L] | 2'FL [g/L] | 3FL [g/L] |
|---|---|---|---|
| FUC/pcBrFucT-HMFT | 3.87 | 0.85 | 1.38 |
| FUC/pBfFucT-HMFT | 0.57 | 4.43 | 0.20 |
| FUC/pHpFutA-HMFT | 0.79 | 2.32 | 0.20 |
| FUC/pcBrFucT-FutC | 0.64 | 0 | 2.45 |
| FUC/pcBrFucT-Te2FT | 0.78 | 0.14 | 2.68 |
| FUC/Ctrl | N.D. | N.D. | N.D. |

As shown in Table 11, it was found that only the FUC/pcBrFucT-HMFT strain accumulated a large amount of LDFT. From the above, it was shown that LDFT can be produced at a high level by combining α1,2-fucosyltransferase HMFT and α1,3-fucosyltransferase cBrFucT.

### [Example 5] Preparation of High-purity LDFT-containing Aqueous Solution

The pH of a culture solution obtained by culturing the Escherichia coli having an LDFT producing ability and constructed in Example 2 in a culture medium was adjusted to 3.0 using sulfuric acid, followed by heating at 70°C for 40 minutes. Subsequently, bacteria cells were removed by centrifugation (4°C, 6000 G, 7000 rpm, 10 minutes), and a supernatant was collected.

The supernatant was passed through a column filled with a cation exchange resin and a column filled with an anion exchange resin in this order to collect a fraction containing LDFT. At this time, DIAION UBK 550 (H⁺ type) (manufactured by Mitsubishi Chemical Group Corporation) as a cation exchange resin and A111S (OH⁻ type) (manufactured by Prolite) as an anion exchange resin were used.

A fraction obtained using a UF membrane module (SIP-0013, manufactured by Asahi Kasei Corporation) was filtered, and the obtained filtrate was concentrated using an evaporator to obtain an LDFT-containing aqueous solution having an LDFT concentration of 400 g/L and HPLC purity of 80.5% (area%).

### [Example 6] Acquisition of Crystalline Solid of LDFT

Using the LDFT-containing aqueous solution obtained in Example 4, an LDFT powder was obtained by a freeze-drying method. To the obtained LDFT powder, a 50% ethanol aqueous solution was added until the powder no longer dissolved. The mixture was left to stand at 4°C for one month to precipitate a crystalline solid. By the same procedure, when an ethanol concentration in the 50% ethanol aqueous solution was 60%, 80%, or 100%, a crystalline solid was also precipitated.

### [Example7] Production of LDFT Crystal by Poor Solvent Addition Method

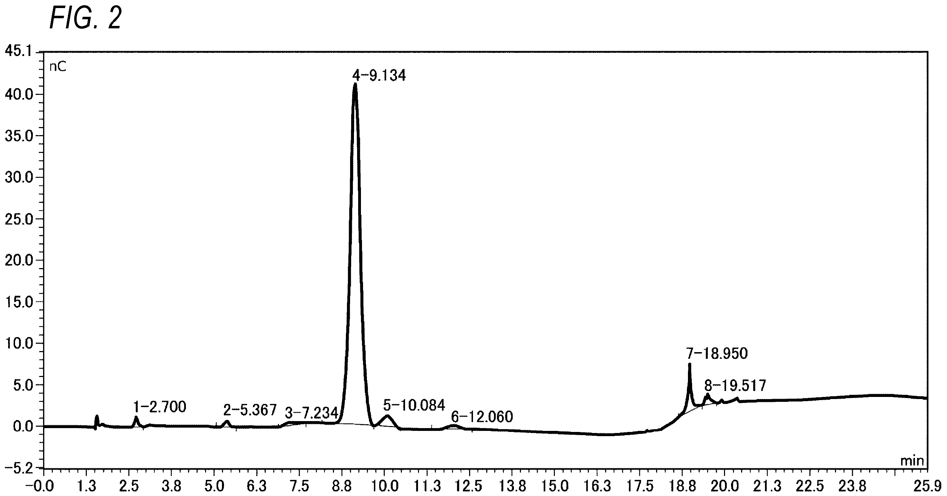

25 mL of the LDFT solution obtained in Example 4 was stirred while being maintained at 40°C, and 30 mL of 100% ethanol as a poor solvent was added dropwise. Subsequently, the crystalline solid obtained in Example 5 was added as a seed crystal to precipitate crystals. The crystals were aged by stirring at room temperature for 24 hours, and then further stirred at 4°C for 72 hours. Thereafter, the crystalline slurry was centrifuged in a basket-type centrifuge (manufactured by Kokusan Co.Ltd.) to obtain 8.7 g of wet crystals. The obtained wet crystals were dried under vacuum at 30°C to obtain 5.2 g of crystals. An LDFT content in the crystalline solid was 85.5% (HPLC purity 92.4%). A chromatogram obtained when the crystal was analyzed by a carbohydrate analyzer ICS-6000 is shown in FIG. 2.

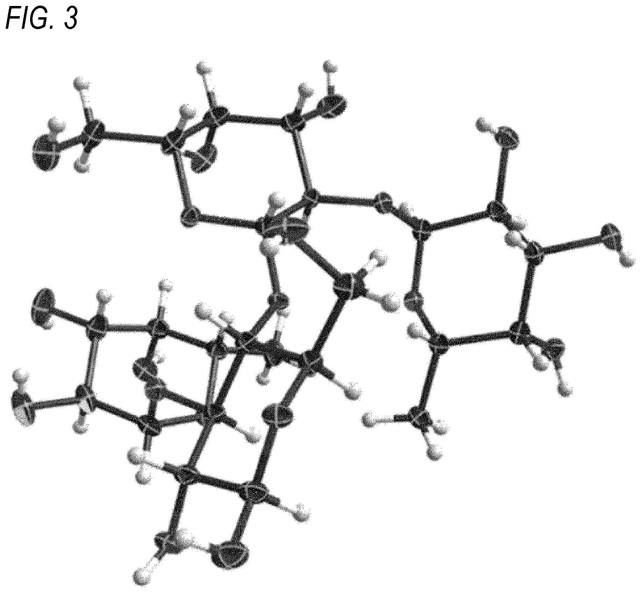

Subsequently, in order to determine the structure of the crystal, single crystal X-ray structure analysis was performed. In analysis of the obtained diffraction data, integration and absorption correction were performed using software (CrysAlis Pro, Rigaku Corporation), and then an initial structure was acquired using SHELX (Acta Crystallogr. Sect. A 2015, 71, 3-8), and the structure was refined using SHELX L (Acta Crystallogr. Sect. C 2015, 71, 3-8). The results are shown in Table 12, and the ORTEP diagram is shown in FIG. 3.

**[Table 12]**

| | |
|---|---|
| Crystal system | Monoclinic |
| Empirical formula | C₂₄H₅₆O₂₆ |
| a (Å) | 8.5351(10) |
| b (Å) | 17.9853(3) |
| c (Å) | 11.9099(10) |
| β (°) | 107.573(10) |
| V (Å³) | 1742.93(4) |
| Measurement temperature (K) | 100.00(10) |
| Z | 2 |
| Space group | P2₁ |
| Rint | 0.0550 |
| R₁ | 0.0403 |
| wR₂ | 0.0952 |
| GoF | 1.030 |
| Flack parameter (Parsons) | -0.029(68) |

As a result of the above analysis, it was confirmed that the crystal had the crystal structure of LDFT and was LDFT•7.2 hydrate having 7.2 water molecules in the unit cell. Subsequently, the crystals were subjected to powder X-ray diffraction measurement. Table 13 shows diffraction angles of peaks with a relative intensity ratio (I/I₀) of 20 or more based on the diffraction results. In the table, "20" represents a diffraction angle (20°), and a "relative intensity" represents a relative intensity ratio (I/I₀).

**[Table 13]**

| 2θ | Relative strength | | 2θ | Relative strength | | 2θ | Relative strength |
|---|---|---|---|---|---|---|---|
| 7.7 | 27 | | 18.7 | 73 | | 26.1 | 36 |
| 9.1 | 59 | | 19.1 | 45 | | 26.9 | 46 |
| 9.7 | 22 | | 19.5 | 49 | | 27.7 | 34 |
| 10.7 | 30 | | 20.0 | 64 | | 28.5 | 51 |
| 11.1 | 100 | | 21.1 | 57 | | 29.1 | 36 |
| 11.8 | 43 | | 21.4 | 53 | | 31.5 | 34 |
| 12.2 | 48 | | 21.7 | 72 | | 32.9 | 33 |
| 12.5 | 36 | | 22.6 | 64 | | 33.3 | 28 |
| 14.5 | 60 | | 22.8 | 72 | | 35.0 | 29 |
| 14.8 | 46 | | 23.0 | 66 | | 36.7 | 31 |
| 15.8 | 96 | | 23.4 | 48 | | 37.5 | 29 |
| 16.6 | 72 | | 24.6 | 31 | | 38.1 | 31 |
| 18.4 | 92 | | 25.4 | 47 | | | |

From the results shown in Table 13, it was confirmed that the obtained LDFT crystal was identical to the LDFT crystal described in Japanese Patent No. 4347042.

Although the present invention has been described in detail with reference to specific embodiments, it is apparent to those skilled in the art that various changes and modifications can be made without departing from the spirit and scope of the present invention. The present application is based on a Japanese Patent Application No. 2022-050799 filed on March 25, 2022, the entire contents of which are incorporated herein by reference. All references cited herein are incorporated in their entirety.

## Claims

1. A microorganism having enhanced activities of a protein according to any one of the following [1] to [3] and a protein according to any one of the following [4] to [6] and improved productivity of lactodifucotetraose (LDFT) as compared with a parent strain,
[1] a protein consisting of the amino acid sequence represented by SEQ ID NO: 8,
[2] a mutant protein having an α1,2-fucosyltransferase activity and consisting of an amino acid sequence in which 1 to 20 amino acids are deleted, substituted, inserted, or added in the amino acid sequence represented by SEQ ID NO: 8,
[3] a homologous protein having an α1,2-fucosyltransferase activity and consisting of an amino acid sequence having an identity of 80% or more with the amino acid sequence represented by SEQ ID NO: 8,
[4] a protein consisting of the amino acid sequence represented by SEQ ID NO: 2,
[5] a mutant protein having an α1,3-fucosyltransferase activity and consisting of an amino acid sequence in which 1 to 20 amino acids are deleted, substituted, inserted, or added in the amino acid sequence represented by SEQ ID NO: 2, and
[6] a homologous protein having an α1,3-fucosyltransferase activity and consisting of an amino acid sequence having an identity of 80% or more with the amino acid sequence represented by SEQ ID NO: 2.

2. A method for producing LDFT, comprising: preparing the microorganism according to claim 1; and producing LDFT in a culture using the microorganism.

3. A method for producing an LDFT crystal from a culture obtained by culturing the microorganism according to claim 1, the method comprising the following steps (i) to (iii):
(i) a step of centrifuging the culture to obtain a supernatant from which bacteria cells are removed;
(ii) a step of subjecting the supernatant obtained in step (i) to cation exchange and anion exchange to obtain a solution by removing ions from the supernatant; and
(iii) a step of obtaining an LDFT crystal from the solution obtained in step (ii).
